# EUROPÄISCHE PATENTSCHRIFT

(11) **EP 1 310 226 B1**
(45) Veröffentlichungstag und Bekanntmachung des Hinweises auf die Patenterteilung: **28.12.2005**
(21) Anmeldenummer: 02024481.0
(22) Anmeldetag: 30.10.2002
(51) Int. Cl.: A61F 13/20, D04H 1/46, D04H 1/02

(54) **Kosmetisches Wattepad**
Cosmetic pad
Tampon cosmétique

(30) Priorität: 08.11.2001 DE 20118212 U; 08.02.2002 DE 20201966 U
(43) Veröffentlichungstag der Anmeldung: 14.05.2003
(73) Patentinhaber: W. Pelz GmbH & Co. KG, 23812 Wahlstedt/Holstein (DE)
(72) Erfinder:
(74) Vertreter: Beetz & Partner

(56) Entgegenhaltungen:
- EP-A- 0 177 277
- EP-A- 0 836 842
- EP-A- 1 106 723
- EP-A- 1 167 605
- WO-A-00/76384

## Beschreibung

Die Erfindung betrifft Wattescheiben aus vorzugsweise gebleichten Baumwollfasern, insbesondere für kosmetische oder hygienische Zwecke, der im Oberbegriff des Ansprüchs 1 angegebenen Gattung.

Zu derartigen Wattescheiben gehören neben weichen lappenförmigen Gebilden insbesondere die weit verbreiteten Wattepads, die in der Kosmetik zum Entfernen von pastösen oder flüssigen Mitteln, beispielsweise beim Abschminken, in der Medizin als sogenannte Tupfer sowie in Labors und im Haushalt zum Abwischen und Aufsaugen von flüssigen und schmierfähigen Medien eingesetzt werden.

Wattepads sind in kreisrunden, rechteckigen oder ovalen Formen von ca. 4 bis 8 cm Durchmesser und etwa 2 bis 4 mm Dicke erhältlich. Sie bestehen aus einem ein- oder mehrlagigen Wirrfaservlies, meist Baumwoll-, Viskose- oder Cellulosefasern, gegebenenfalls mit bestimmten Anteilen an anderen Faserarten. Zur Erzielung bzw. Förderung bestimmter Effekte können die Wattepads mit besonders geeigneten Additiven versehen sein, beispielsweise mit Lösungsmitteln für Schminke, mit entzündungshemmenden und/oder blutstillenden Substanzen oder dergleichen.

Wesentliche Kriterien von Wattescheiben sind eine möglichst hohe Saug- und Aufnahmefähigkeit für Flüssigkeiten, damit diese in ausreichenden Mengen von beliebigen Unterlagen, insbesondere auch von der Haut, durch Betupfen oder Abstreifen in das Vliesinnere gelangen und dort tropffrei gehalten werden. Wichtig sind ferner gute Adhäsionseigenschaften in den Oberflächenbereichen gegenüber pastösen Massen. Beispielsweise beim Abschminken sollen möglichst große Mengen an Schminke, Creme od. dgl. an der jeweiligen Oberfläche des Wattepads haften bleiben, damit der Abschminkvorgang schnell und mit möglichst wenigen Wattepads durchgeführt werden kann. Des weiteren müssen die aus einem relativ losen Faservlies aufgebauten Wattescheiben eine ausreichende Reißfestigkeit besitzen, um überhaupt gebrauchsfähig zu sein und Rißbildungen bei den verschiedenartigen Anwendungen zu vermeiden. Schließlich sollen die Wattepads auch weitgehend fusselfrei sein, damit beim Abstreifen einer Flüssigkeit oder pastösen Masse von einer relativ rauhen Oberfläche, beispielsweise von behaarter Haut, keine Fasern oder Faserbüschel aus dem Faserverbund herausgezogen werden und an dieser Oberfläche haften bleiben.

Aus der EP 1 106 723 A1 sind Wattescheiben aus hydrophiler Baumwolle für die Hautpflege bekannt, die ein Flächengewicht von mindestens 150 g/m² haben, und deren Reißfestigkeit zumindest 20 N in Laufrichtung und zumindest 16 N in Querrichtung betragen soll. Die beiden Außenseiten dieser Wattescheiben sind unterschiedlich strukturiert, wobei die eine Außenseite die üblichen parallelen Feinrillen in einem Zwischenabstand von 0,4 bis 1,2 mm aufweist, die in herkömmlicher Weise durch Wasservernadelung gebildet worden sind. Die andere Außenseite hat vertiefte Rillen in einem Zwischenabstand von 1,0 bis 8,0 mm und einer Rillentiefe von mehr als 0,25 mm. Zumindest 50 % der Baumwollfasern einer Wattescheibe sollen miteinander verbunden sein. Diese Wattescheiben erfüllen die Forderungen bezüglich Saug- und Aufnahmefähigkeit für Flüssigkeiten, Adhäsionsfähigkeit in den Oberflächenbereichen für pastöse Massen und auch Reißfestigkeit. Probleme ergeben sich allerdings durch die Neigung dieser bekannten Wattepads zum Fusseln, wenn der Abstand zwischen den vertieften Rillen im Bereich von 6 bis 8 mm liegt. Da die Zonen zwischen benachbarten vertieften Rillen nicht durch geeignete Maßnahmen verfestigt sind, liegt zumindest ein Teil der Baumwollfasern in einem relativ losen Verbund in den oberflächennahen Schichten, so daß Fasern beim Entlangstreichen auf einer rauheren Oberfläche herausgelöst werden und an dieser haften bleiben. Aus diesem Grunde sollen die Zwischenabstände zwischen den vertieften Rillen vorzugsweise zwischen 2,0 und 4,0 mm liegen.

Ferner ist aus der CH 672 249 A5 eine Wattescheibe in runder oder eckiger Form bekannt, die zwei verdichtete Oberflächen zwischen einem unverdichteten Körper aufweist. Diese Wattescheibe wird zum Auftragen und/oder Aufsaugen von flüssigen oder pastösen Medien in der Kosmetik, der Medizin und anderen technischen Gebieten eingesetzt. Das Verdichten der beiden oberflächennahen Schichten soll ein übermäßiges Fusseln verhindern. Es erfolgt durch Kalandrieren zwischen profilierten Walzen bei erhöhten Temperaturen von 100 bis 200 °C. Die Oberflächen der so hergestellten Wattescheiben tragen voneinander unterschiedliche Waffelmuster, wobei die Einsenkungen des gröberen Waffelmusters eine gewisse Depotbildung der abgesteiften Pasten und die erhabenen Stellen eine intensivierte Saugwirkung ermöglichen sollen.

Aus der EP 0 405 043 A sind Wattescheiben zum Auftragen oder Aufsaugen von flüssigen oder halbfesten Substanzen bekannt, die zwei flächenhaft verdichtete Außenschichten aufweisen, zwischen denen sich eine Innenschicht aus unverdichtetern Fasermaterial befindet. Die Ränder dieser drei Schichten sind untereinander verbunden. Zum Erhalt von Depoträumen für pastöse oder flüssige Substanzen sind zumindest in einer der verfestigten Außenschichten geradlinige Vertiefungen durch Prägen ausgebildet. Nachteilig bei diesen bekannten Wattescheiben ist die lediglich auf die Oberfläche beschränkte Verfestigung, was auf deren Herstellung durch Prägen zurückzuführen ist. Eine Verfilzung der oberflächennahen Faserschichten kann durch Prägen zwischen Druckwerkzeugen nicht erhalten werden. Weitere Wattepads mit strukturierten Oberflächen sind in den Druckschriften EP 0 750 062 B1, FR 2 052 089 und FR 2 502 470 beschrieben.

Aufgabe der Erfindung ist es, für verschiedenartige Anwendungszwecke ausreichend feste Wattescheiben von hoher Saug- und Adhäsionsfähigkeit zu schaffen, die ein effektives Abstreifen pastöser Massen von der Unterlage ohne Fusseln sowie eine effektive Depotwirkung für die abgestreiften Massen besitzen.

Zur lösung dieser Aüfgabe zeichnen sich erfindungsgemäße Wattescheiben, durch die in den unabhängigen Ansprüchen 1, 5 und 9 jeweils angegebenen Merkmale aus.

In beiden Oberseiten der Wattescheiben können die durch Wasservernadelung gebildeten parallelen Feinrillen von 0,1 bis 0,2 mm Tiefe und 0,5 bis 1,5 mm Zwischenabstand ausgebildet sein. Diese Feinrillen werden vorteilhaft in einem ersten Vernadelungsvorgang in den beiden Oberflächen geformt, so daß die in einem nachfolgenden zweiten Vernadelungsvorgang mit den Rippen, Einsenkungen und/oder Breitrillen versehenen Oberflächen diese Feinrillen enthalten. Diese Feinrillen ergeben aufgrund der durch die Wasservernadelung erzielten Faserverfilzung in den Oberflächenbereichen gegenüber unverfestigten oder geprägten Oberflächen eine wesentlich verminderte Fusselneigung, da die Fasern in diesen Oberflächenbereichen mehrfach eingebunden sind. Der geringe Zwischenabstand zwischen den Feinrillen gewährleistet eine effektive Einbindung aller Baumwollfasern. Die geringe Tiefe und die relativ kleinen Zwischenabstände der Feinrillen ergeben einen weitgehend gleichmäßigen Festigkeitsanstieg in jeder der beiden Oberflächenschichten mit jeweils hoher Saug- und Aufnahmefähigkeit für Flüssigkeiten. Durch Zusammenwirken von mehreren Effekten der erfindungsgemäßen Gestaltung der Oberflächenbereiche haben die neuen Wattescheiben gegenüber herkömmlichen Ausführungen eine erheblich vergrößerte Verbundfestigkeit und ein exzellentes Speichervermögen für pastöse Massen.

Die zusätzlich in eine oder auch beide Oberflächen durch Wasservernadelung geformten vorstehenden Rippen, Einsenkungen und/oder Breitrillen wirken mit den Feinrillen in der Weise zusammen, daß durch letztere eine zusätzliche Verfestigung der vorstehenden Rippen und der rillennahen Umgebungszonen der Breitrillen erzielt wird. Durch die erfindungsgemäß ausgebildeten prägnanten Rippen wird ferner eine wischerblattähnliche Abstreifwirkung von flüssigen und pastösen Substanzen erzielt, wenn die Abstreifrichtung quer zu den jeweiligen Rippen verläuft. Zusätzlich ergibt sich ein ausgeprägter Depoteffekt für die pastösen Massen, die sich in den eingesenkten Bereich vor und hinter den Rippen sowie in den Breitrillen und den Einsenkungen ansammeln. Auf diese Weise können größere Schminkmengen bei einer Abstreifbewegung vom Wattepad aufgenommen werden.

Da sich der von den durch Wasservernadelung ausgebildeten Feinrillen erzielte Verfestigungseffekt nur auf relativ dünne Oberflächenschichten beschränkt und die Fasern in den Kernschichten der Wattescheibe in losem Verbund vorliegen, haben die erfindungsgemäßen Wattescheiben eine hohe und über die Fläche weitgehend gleichmäßige Saugfähigkeit sowie eine besonders gute Speicherwirkung für Flüssigkeiten in den Kernbereichen der Scheibe.

Die erfindungsgemäßen Wattescheiben können aus einer Vielzahl von geeigneten Faserarten bestehen, beispielsweise aus gebleichten Baumwollfasern, Cellulosefasern, Viskosefasern, synthetischen Fasern, Bikomponentenfasern sowie aus deren Gemischen.

Das zur Herstellung der Wattescheiben erzeugte Wirrfaservlies oder die Wattescheiben selbst können mit geeigneten Additiven versetzt sein, um bestimmte Eigenschaften zu erzielen.

Weiter Vorzüge und Besonderheiten der Erfindung ergeben sich aus der folgenden Beschreibung von bevorzugten Ausführungsbeispielen anhand der Zeichnung. Es zeigen:
- Fig. 1: eine erste Ausführung einer an einer Oberseite mit parallelen geradlinigen Rippen versehenen Wattescheibe in vergrößertem perspektivischem Ausschnitt;
- Fig. 2: eine andere Ausführung einer Wattescheibe mit an beiden Oberseiten ausgebildeten Längsrippen in vergrößertem perspektivischem Ausschnitt;
- Fig. 3: einen oberen Bereich eines Wattepads mit in versetzten Reihen angeordneten Einsenkungen in vergrößertem perspektivischen Ausschnitt;
- Fig. 4a bis f: mehrere Wattescheiben der Typen nach Fig. 1 bis 3 mit unterschiedlichen flächigen Mustern in Draufsicht;
- Fig. 5: eine weitere Ausführung einer Wattescheibe mit beidseitigen Feinrillen und an einer Oberseite ausgebildeten Breitrillen;
- Fig. 6a, b: jeweils eine oberflächennahe Schicht einer Wattescheibe mit zwei unterschiedlich ausgebildeten Breitrillen in vergrößertem Querschnitt;
- Fig. 7 bis 9: Wattescheiben vom Typ nach Fig. 4 mit unterschiedlichen Breitrillenmustern.

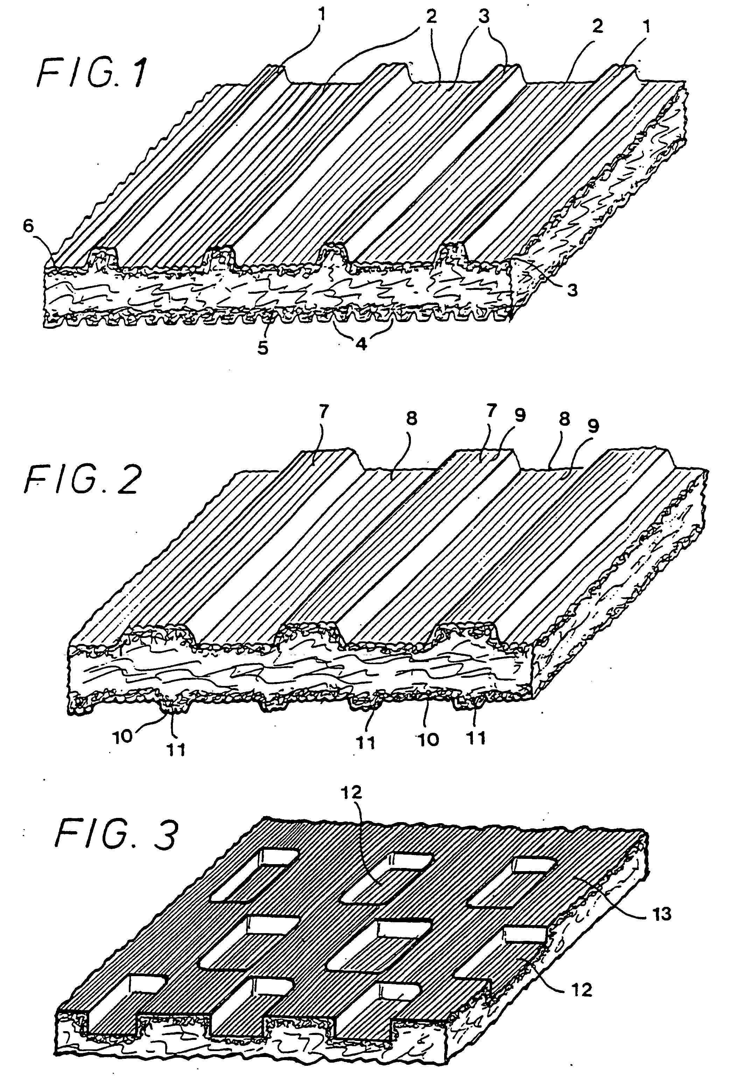

Die in Fig. 1 dargestellte Wattescheibe kann für verschiedene Anwendungen großflächig (z.B. Lappen, Tücher, Bestandteil von Damenbinden oder dergleichen) sein oder als sogenanntes Wattepad eine kreisrunde oder ovale Form haben. Dieses Wattepad besteht zumindest zum überwiegenden Teil aus gebleichten Baumwollfasern und hat eine herkömmliche Dicke von 2 bis 4 mm. Wie gezeigt, weist dieses Wattepad an seiner Oberseite mehrere parallele Längsrippen 1 auf, deren Breite etwa der Rippenhöhe entspricht. Die durch Wasservernadelung abgesenkten Bereiche 2 zwischen benachbarten Rippen 1 sind bei dieser Ausführung etwa doppelt so breit wie die Rippen 1. Zusätzlich zu den Rippen sind an der gesamten Oberseite des Wattepads eine Vielzahl von zu den Rippen 1 parallel verlaufenden Feinrillen 3 gebildet, die einen geringen Zwischenabstand von etwa 0,5 mm sowie eine Rillentiefe von etwa 0,1 mm haben. Diese nach dem bekannten Wasservernadelungsverfahren hergestellten Feinrillen 3 befinden sich auf den Rippen 1 ebenso wie auf den abgesenkten Zwischenbereichen 2. Wie dargestellt, wird durch die oberseitige Wasservernadelung eine intensive Verfilzung der Baumwollfasern in der oberflächennahen Faserschicht 6 erzielt, was eine erhöhte Reißfestigkeit sowie auch eine festere Einbindung der Einzelfasem und damit eine verringerte Fusselwirkung ergibt. Um diese Effekte zu verstärken, ist auch die Unterseite des Wattepads mit einer Vielzahl von Feinrillen 4 versehen, deren Tiefe und Zwischenabstand hier allerdings größer als diejenigen der oberseitigen Feinrillen 3 gewählt sind. Auch diese Feinrillen 4 sind durch Wasservernadelung hergestellt, um die dadurch erreichbare Verfilzung der Bodenschicht 5 in ähnlicher Weise wie bei der oberseitigen Faserschicht 6 zu erhalten.

Das Ausführungsbeispiel nach Fig. 2 entspricht in seinem Grundaufbau der Ausführung nach Fig. 1. Allerdings haben hier die oberseitigen Rippen 7 etwa die gleiche Breite wie die dazwischenliegenden abgesenkten Bereiche 8, wobei die Oberseite der Rippen 7 und auch der abgesenkten Bereiche 8 zur Erzielung des vorgenannten Verfilzungseffektes der oberflächennahen Baumwollfasern mit parallelen Feinrillen 9 versehen sind. An der Unterseite dieses Wattepads sind zusätzlich zu Feinrillen 10 mehrere Rippen 11 ausgebildet, deren Höhe und Breite bei dieser Ausführung kleiner als die entsprechenden Werte der oberseitigen Rippen 7 sind.

Die in Fig. 3 dargestellte Wattescheibe weist vorzugsweise durch Wasservernadelung erzeugte oberseitige Einsenkungen 12 auf, die in gegeneinander versetzten Reihen angeordnet sind und eine den Rippen ähnliche Funktion hinsichtlich Abstreifwirkung und Deponierung erfüllen. Auch diese Ausführung besitzt die jeweils durchgehenden Feinrillen an ihrer gesamten Oberseite, um das unerwünschte Fusseln zu unterbinden und die Reißfestigkeit der Wattescheibe zu erhöhen. Die erhabenen Bereiche 13 zwischen den Einsenkungen 12 haben eine den Rippen der Ausführung nach Fig. 1, 2 ähnliche Abstreiffunktion.

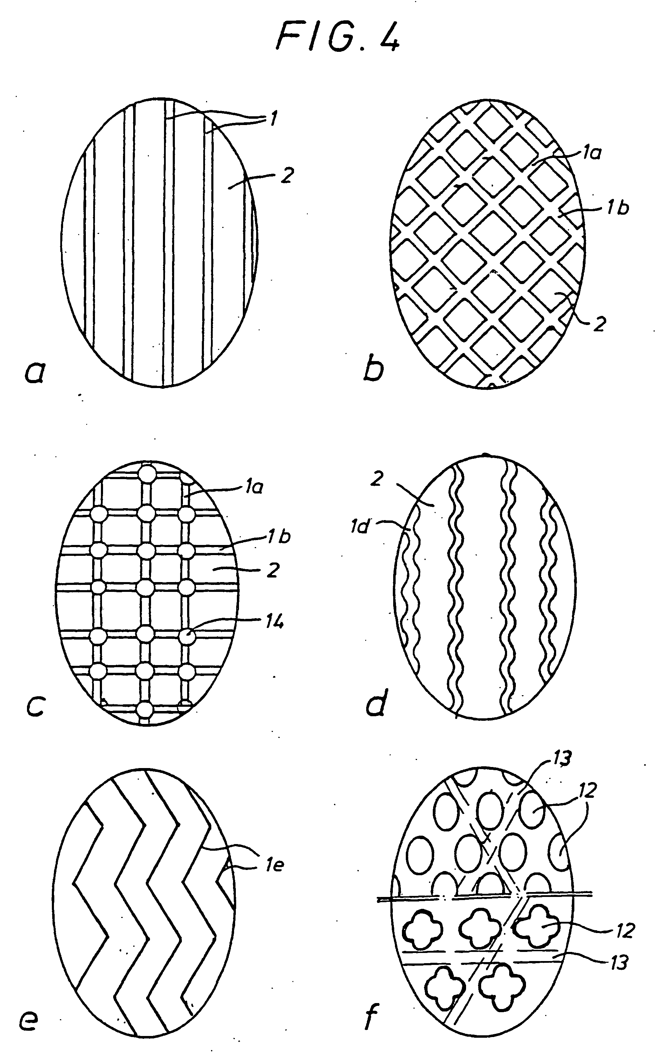

In Fig. 4a bis f sind ovale Wattepads der in Fig. 1 gezeigten Ausführung mit verschiedenen Rippenmustern und längs, quer oder schräg verlaufenden - nicht dargestellten - Feinrillen in Draufsicht dargestellt. Das Wattepad nach Fig. 4a hat parallele Längsrippen 1 und entspricht dem Typ nach Fig. 1 bzw. 2. Bei der Ausführung nach Fig. 4b sind zwei Rippenscharen 1a, 1b rechtwinklig zueinander unter jeweils 45°-Winkeln zur Längsrichtung vorgesehen, wodurch sich ein waffelartiges Muster ergibt. Auch die Ausführung nach Fig. 4c weist zwei Scharen von zueinander senkrechten Rippen 1a, 1b auf, die hier jedoch in Längs- und Querrichtung verlaufen, wobei in den Schnittpunkten der Rippen etwa kreisförmige vorstehende Noppen 14 ausgebildet sind. Die Ausführung nach Fig. 4d entspricht im wesentlichen derjenigen nach Fig. 4a, wobei hier allerdings die Rippen 1d einen wellenförmigen Verlauf haben. Auch diese wellenförmigen Rippen 1d können kreuzweise angeordnet werden, wodurch sich ein der Fig. 4b ähnliches Waffelmuster ergibt.

Das in Fig. 4e dargestellte Wattepad hat an einer Oberfläche zick-zack-förmige Längsrippen 1e. Wie in Fig. 4f gezeigt, können die Einsenkungen 12 verschiedene geometrische Konturen haben, wobei die zwischen den Einsenkungen 12 vorhandenen Bereiche 13 - wie durch Strichlinien angedeutet - schräg verlaufende Rippenscharen darstellen.

Die in den Fig. 4a bis f dargestellten Muster können nur an einer Außenseite des Wattepads oder auch an dessen beiden Außenseiten in jeweils geeigneter Kombination vorgesehen werden.

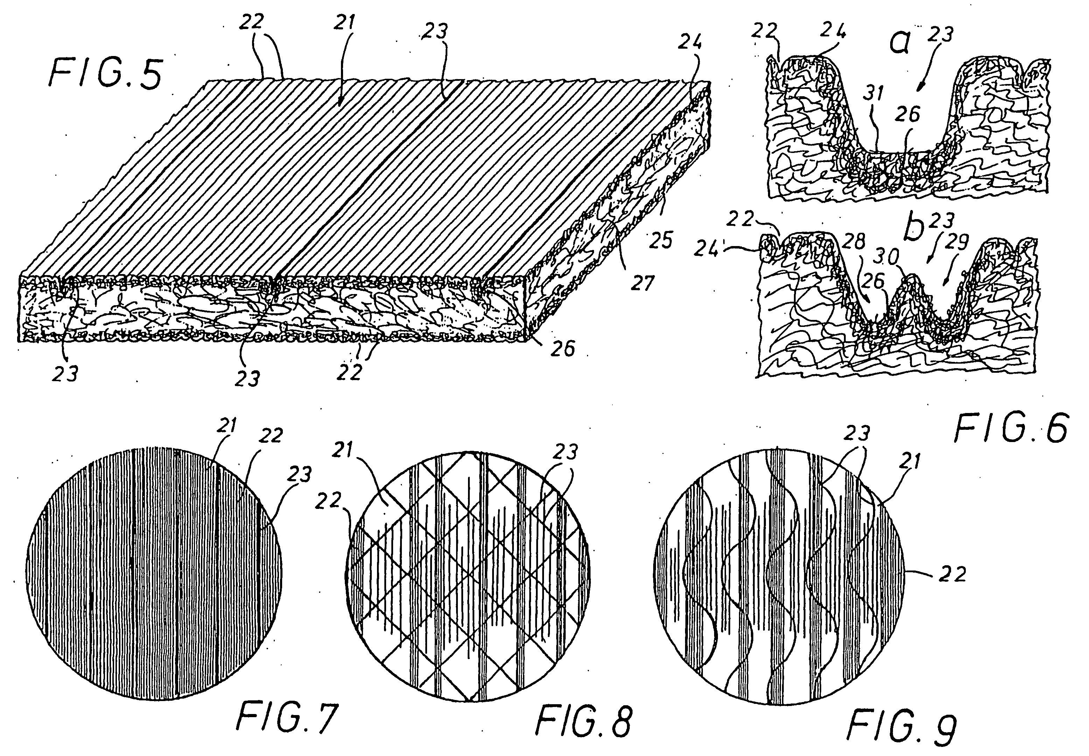

Die in Fig. 5 in perspektivischem Ausschnitt dargestellte Wattescheibe hat eine Ober- bzw. Außenseite 21 mit einer Vielzahl von zueinander parallelen Feinrillen 22, die durch Wasservernadelung gebildet sind und einen Zwischenabstand von 0,5 bis 0,7 mm sowie eine Rillentiefe von 0,1 bis 0,2 mm haben. Durch die Feinheit und die Dichte dieser Rillen wird der visuelle Eindruck einer gleichmäßigen Oberfläche vermittelt. Zusätzlich zu diesen Feinrillen 22 sind in der Oberseite der dargestellten Wattescheibe Breitrillen 23 gebildet, die hier parallel zueinander und zu den Feinrillen verlaufen. Diese Breitrillen 23 sind in ähnlicher Weise wie die Feinrillen 22 durch Wasservernadelung des ursprünglich aus losen Baumwoll- bzw. Cellulosefasern bestehenden Wirrfaservlieses geformt. Die Rillentiefe dieser Breitrillen 23 beträgt etwa 0,3 bis 0,8 mm und ihr Zwischenabstand liegt hier bei etwa 9,0 mm. Der Wattepad selbst hat eine Dicke von etwa 2, 5 bis 3 mm und kann ein Flächengewicht von etwa 100 bis 280 g/m² haben.

Wie insbesondere aus den Fig. 5 und 6 ersichtlich, sind die Baumwollfasern in den beiden oberflächennahen Schichten 24, 25 des Wattepads durch die Wirkung der Wasservernadelung intensiv miteinander verschlungen, wodurch sich eine wesentlich gesteigerte Zug- und Reißfestigkeit dieser beiden oberflächennahen Schichten 24, 25 ergibt. Ein erhöhter Verschlingungseffekt der Fasern ist auch im unmittelbaren Umgebungsbereich 26 der Breitrillen 23 vorhanden, wobei sich diese Verschlingungsbereiche 26 tiefer in den im wesentlichen aus einem losen und extrem saugfähigen Faserverbund bestehenden Vlieskern 27 erstrecken. Die vermehrte Einbindung der Fasern in den Oberflächenschichten 24, 25 verhindert ein Herauslösen von Einzelfasern und damit das Fusseln auch nach wiederholten Abstreifbewegungen der Wattescheibe auf relativ rauhern Untergrund sowie auch ein unerwünschtes Austropfen von aufgenommenen Flüssigkeiten.

In den Figuren 6a und 6b sind zwei besondere Ausführungen der Breitrillen 23 gezeigt, wobei die Breitrille nach Fig. 6a durch einen verbreiterten Wasserstrahl mit hohem Druck von über 40 bar geformt ist und einen ausgeprägten Rillengrund 11 aufweist, während es sich bei der Breitrille 23 der Fig. 6b um eine "Doppelrinne" handelt, die durch zwei dicht nebeneinander liegende Druckwasserstrahlen von z.B. bis 40 bar erzeugt wurde und zwei seitliche Vertiefungen 28, 29 sowie einen mehr oder weniger ausgeprägten Mittelsteg 30 aufweist. Wesentlich bei beiden Ausführungen sind die intensiviert verschlungenen Faserbereiche 26 in den angrenzenden Faserzonen und das durch die vergrößerten Querschnitte dieser Breitrillen 23 erhöhte Aufnahmevermögen für pastöse Massen, insbesondere Schminke.

Bei den in den Figuren 7 bis 9 dargestellten Wattescheiben handelt es sich ebenfalls um Wattepads, deren beide Oberseiten in gleicher Weise wie bei der Ausführung nach Fig. 4 mit den parallelen Feinrillen 21 versehen sind, von denen jedoch nur jeweils einige dargestellt sind. Die sichtbare Oberseite jedes Wattepads weist Breitrillen 23 auf, die gemäß Fig. 5 etwa V-förmig oder gemäß Fig. 6a, 6b ausgebildet sein können. Bei der Ausführung nach Fig. 8 sind diese Rillen 23 jeweils kreuzweise unter 45°-Winkeln und bei der Ausführung nach Fig. 9 wellenförmig angeordnet. Auf der - nicht sichtbaren - Unterseite dieser beiden Wattepads sind Feinrillen in gleichen oder auch anderen Zwischenabständen wie auf der Oberseite durch Wasservernadelung ausgebildet. Daneben können auf diesen Unterseiten auch Breitrillen in gleichen oder einem anderen Muster wie auf der Oberseite vorgesehen sein.

Die Herstellung der Feinrillen und erfindungsgemäßen Rippen in den verschiedenartigen Konturen erfolgt zweckmäßig durch ein mehrstufiges Wasservernadelungsverfahren, und zwar unter Verwendung von geeigneten z.B. mitlaufenden Schablonen. Diesbezügliche Verfahren gehören seit langem zum Stand der Technik und sind u.a. in den Druckschriften EP 0 177 277 B1, EP 0 777 782 B1, JP 5-239 753 A beschrieben.

Die erfindungsgemäßen Wattescheiben können aus einer Vielzahl von geeigneten Faserarten, beispielsweise aus gebleichten Baumwollfasern, Cellulosefasern, Viskosefasern, synthetischen Fasern, Mehrkomponentenfasern sowie aus deren Gemischen, bestehen und haben ein Flächengewicht von 150 bis 400 g/m².

Bei dem Ausführungsbeispiel nach Fig. 5 bis 9 können die Breitrillen 23 in den dargestellten Mustern oder in anderem Verlauf auch in den beiden Oberflächen der Wattescheibe eingeformt sein, wodurch sich die Depotwirkungen für fließfähige bzw. pastöse Massen bei beiden Oberseiten ergibt. Ferner können die Feinrillen 22 in den beiden Oberseiten gleiche oder in vorgegebenen Grenzen verschiedene Tiefen und Zwischenabstände haben. Ferner ist auch ein zick-zack-förmiger Verlauf der Feinrillen 22 und insbesondere der Breitrillen 23 möglich, um einerseits die Festigkeitssteigerung in mehreren Zugrichtungen zu erzielen und daneben auch eine erhöhte und von der Abstreifrichtung unabhängigere Depotwirkung zu erhalten.

Für Wattepads, bei denen die Breitrillen nur an einer Oberseite vorhanden sind, ist es zweckmäßig, die Breitrillen farbig auszuführen, um anzuzeigen, daß diese Oberseite eine von der feingerillten Gegenseite verschiedene Wirkung, nämlich eine intensivierte Abstreifwirkung sowie einen Depoteffekt, besitzt. Zu diesem Zweck kann auch die eine oder andere Oberseite des Wattepads mit besonderen Markierungen, wie einer zonenweise oder großflächigen Färbung, Farbtupfern oder dergleichen versehen sein, die beispielsweise durch eine Drucktechnik aufgebracht werden können.

## Patentansprüche

1. Wattescheibe aus vorzugsweise gebleichten Baumwollfasern, insbesondere für kosmetische oder hygienische Zwecke,
- die an zumindest einer Oberseite durch Wasservernadelung gebildete parallele Feinrillen (3, 9) von 0,1 bis 0,2 mm Tiefe und 0,5 bis 1,5 mm Zwischenabstand aufweist,
**dadurch gekennzeichnet, dass**
- an zumindest einer der die Feinrillen (3, 9) aufweisenden Oberseite durch einen nachfolgenden zweiten Wasservernadelungsvorgang geformte Rippen (1, 7) ausgebildet sind,
- die Rippen (1, 7) eine Breite von etwa 1,0 bis 10,0 mm, eine Höhe von 0,4 bis 2,5 mm und einen Zwischenabstand von 2,0 bis 10,0 mm haben,
- wobei die Rippen (1, 7) und die eingesenkten Bereiche (2, 8) zwischen benachbarten Rippen (1, 7) die oberseitigen Feinrillen (3, 9) aufweisen.

2. Wattescheibe nach Anspruch 1,
**dadurch gekennzeichnet, dass**
beide Oberseiten die Feinrillen (3, 4) und nur eine der Oberseiten die Rippen (1) aufweisen.

3. Wattescheibe nach Anspruch 1,
**dadurch gekennzeichnet, dass**
an beiden Oberseiten Rippen (7, 11) ausgebildet sind, wobei die Rippen (7) an der einen Oberseite von den Rippen (11) an der anderen Oberseite hinsichtlich Form und Verlauf verschieden sein können.

4. Wattescheibe nach einem der vorstehenden Ansprüche,
**dadurch gekennzeichnet, dass**
die Rippen (1a, 1b; 1d; 1e) einen durchgehenden oder unterbrochenen gradlinigen, wellenförmigen, parallelen oder sich kreuzenden Verlauf haben.

5. Wattescheibe aus vorzugsweise gebleichten Baumwollfasern insbesondere für kosmetische oder hygienische Zwecke,
- die an zumindest einer Oberseite durch Wasservernadelung gebildete parallele Feinrillen (3, 9) von 0,1 bis 0,2 mm Tiefe und 0,5 bis 1,5 mm Zwischenabstand aufweist,
**dadurch gekennzeichnet, dass**
an zumindest einer der die Feinrillen aufweisenden Oberseiten durch einen nachfolgenden zweiten Wasservernadelungsvorgang geformte runde oder mehreckige Einsenkungen (12) von 0,5 bis 2,5 mm Tiefe und 3,0 bis 10,0 mm Durchmesser ausgebildet sind.

6. Wattescheibe nach Anspruch 5,
**dadurch gekennzeichnet, dass**
beide Oberseiten die Feinrillen und nur eine der Oberseiten die Einsenkungen (12) aufweisen.

7. Wattescheibe nach Anspruch 5,
**dadurch gekennzeichnet, dass**
auf beiden Oberseiten verschiedenartige Einsenkungen (12) ausgebildet sind.

8. Wattescheibe nach Anspruch 5,
**dadurch gekennzeichnet, dass**
an der von den Einsenkungen (12) freien anderen Oberseite Feinrillen (3) und Rippen (1, 7) nach einem der Ansprüche 1 oder 4 ausgebildet sind.

9. Wattescheibe aus vorzugsweise gebleichten Baumwollfasern insbesondere für kosmetische oder hygienische Zwecke,
- die an zumindest einer Oberseite (21) durch Wasservernadelung gebildete parallele Feinrillen (22) von 0,1 bis 0,2 mm Tiefe und 0,5 bis 0,7 mm Zwischenabstand aufweist,
**dadurch gekennzeichnet, dass**
- an zumindest einer der die Feinrillen (22) aufweisenden Oberseite (21) durch einen nachfolgenden zweiten Wasservernadelungsvorgang geformte zusätzliche Breitrillen (23) von etwa 0,3 bis 0,8 mm Tiefe und 9,0 bis 15,0 mm Zwischenabstand ausgebildet sind.

10. Wattescheibe nach Anspruch 9,
**dadurch gekennzeichnet, dass**
jede Breitrille (23) durch einen verbreiterten Druckwasserstrahl geformt ist und einen ausgeprägten Rillengrund (31) aufweist.

11. Wattescheibe nach Anspruch 9,
**dadurch gekennzeichnet, dass**
jede Breitrille (23) durch zwei nebeneinander vorgesehene Druckwasserstrahlen geformt ist und im Rillengrund zwei seitliche Vertiefungen (28, 29) sowie einen Mittelsteg (30) aufweist.

12. Wattescheibe nach einem der Ansprüche 9 bis 11,
**dadurch gekennzeichnet, dass**
beide Oberseiten die Feinrillen (22) und nur eine der Oberseiten die Breitrillen (23) aufweisen.

13. Wattescheibe nach einem der Ansprüche 9 bis 11,
**dadurch gekennzeichnet, dass**
beide Oberseiten mit durchlaufenden oder unterbrochenen Breitrillen (23) versehen sind, die unterschiedliche Zwischenabstände haben oder gegeneinander versetzt angeordnet sind.

14. Wattescheibe nach einem der Ansprüche 9 bis 13,
**dadurch gekennzeichnet, dass**
die Breitrillen (23) zueinander und zu den Feinrillen (22) parallel angeordnet sind.

15. Wattescheibe nach einem der Ansprüche 9 bis 14,
**dadurch gekennzeichnet, dass**
die Breitrillen (23) in zwei sich kreuzenden Rillenscharen unter jeweils spitzen Winkeln zu den Feinrillen (22) angeordnet sind.

16. Wattescheibe nach einem der Ansprüche 9 bis 15,
**dadurch gekennzeichnet, dass**
die Breitrillen (23) bogen-, zick-zack- oder wellenförmig verlaufen.

17. Wattescheibe nach einem der Ansprüche 9 bis 16,
**dadurch gekennzeichnet, dass**
das Muster der Breitrillen (23) auf der einen Oberseite von dem Muster der Breitrillen auf der anderen Oberseite verschieden ist.

18. Wattescheibe nach einem der vorstehenden Ansprüche,
**dadurch gekennzeichnet, dass**
die beiden Oberseiten unterschiedliche Farben haben.

19. Wattescheibe nach einem der vorstehenden Ansprüche,
**dadurch gekennzeichnet,**
**dass** zumindest eine der Oberseiten mit Markierungen versehen ist.

20. Wattescheibe nach einem der vorstehenden Ansprüche,
**dadurch gekennzeichnet, dass**
die Zwischenabstände und Tiefen der Feinrillen (2, 22) auf der einen Oberseite von den Zwischenabständen und Tiefen der Feinrillen (4, 10) auf der anderen Oberseite verschieden sind.

## Claims

1. Disc-shaped cotton pad made from preferably bleached cotton fibres, in particular for cosmetic or hygienic purposes,
- which has parallel fine striae (3, 9) of 0.1 to 0.2 mm depth and 0.5 to 1.5 mm intermediate distance formed through water needling on at least one upper side,
**characterised in that**
- ribs (1, 7) formed through a subsequent second water needling process are arranged on at least one of the upper sides having the fine striae (3, 9),
- the ribs (1, 7) have a width of about 1.0 to 10.0 mm, a height of 0.4 to 2.5 mm and an intermediate distance of 2.0 to 10.0 mm,
- wherein the ribs (1, 7) and the depressed regions (2, 8) between adjacent ribs (1, 7) have the upper side fine striae (3, 9).

2. Disc-shaped cotton pad according to claim 1,
**characterised in that**
the two upper sides have the fine striae (3, 4) and only one of the upper sides has the ribs (1).

3. Disc-shaped cotton pad according to claim 1,
**characterised in that**
ribs are formed on both upper sides (7, 11) wherein the ribs (7) on one upper side can differ in form and pattern from the ribs (11) on the other upper side.

4. Disc-shaped cotton pad according to one of the preceding claims,
**characterised in that**
the ribs (1a; 1b; 1d; 1e) have a continuous or interrupted straight line, wave form, parallel or crossing pattern.

5. Disc-shaped cotton pad made from preferably bleached cotton fibres, in particular for cosmetic or hygienic purposes,
- which has parallel fine striae (3, 9) of 0.1. to 0.2 mm depth and 0.5 to 1.5 mm intermediate distance formed through water needling on at least one upper side,
**characterised in that**
round or multi-corner troughs (12) of 0.5 to 2.5 mm depth and 3.0 to 10.0 mm diameter formed through a subsequent second water needling process are formed on at least one of the upper sides having the fine striae.

6. Disc-shaped cotton pad according to claim 5,
**characterised in that**
the two upper sides have the fine striae and only one of the upper sides has the troughs (12).

7. Disc-shaped cotton pad according to claim 5,
**characterised in that**
different types of troughs (12) are formed on the two upper sides.

8. Disc-shaped cotton pad according to claim 5,
**characterised in that**
fine striae (3) and ribs (1, 7) are formed according to one of the claims 1 or 4 on the other upper side free of the troughs (12).

9. Disc-shaped cotton pad made from preferably bleached cotton fibres, in particular for cosmetic or hygienic purposes,
- which has parallel fine striae (22) of 0.1 to 0.2 mm depth and 0.5 to 0.7 mm intermediate distance formed through water needling on at least one upper side (21),
**characterised in that**
- additional wide striae (23) of about 0.3 to 0.8 mm depth and 9.0 to 15.0 mm intermediate distance formed through a subsequent second water needling process are formed on at least one of the upper sides (21) having the fine striae (22).

10. Disc-shaped cotton pad according to claim 9,
**characterised in that**
each wide stria (23) is formed by means of a widened pressurised water jet and has a distinct stria bottom (31).

11. Disc-shaped cotton pad according to claim 9,
**characterised in that**
each wide stria (23) is formed by means of pressurised water jets provided beside each other and has two lateral troughs (28, 29) and a middle web (30) in the bottom of the stria.

12. Disc-shaped cotton pad according to one of the claims 9 to 11,
**characterised in that**
the two upper sides have the fine striae (22) and only one of the upper sides has the wide striae (23).

13. Disc-shaped cotton pad according to one of the claims 9 to 11,
**characterised in that**
the two upper sides are provided with continuous or interrupted wide striae (23) which have different intermediate distances or are arranged offset against each other.

14. Disc-shaped cotton pad according to one of the claims 9 to 13,
**characterised in that**
the wide striae (23) are arranged parallel to each other and to the fine striae (22).

15. Disc-shaped cotton pad according to one of the claims 9 to 14,
**characterised in that**
the wide striae (23) are arranged in two crossing groups of striae at acute angles to the fine striae (22).

16. Disc-shaped cotton pad according to one of the claims 9 to 15,
**characterised in that**
the wide striae (23) run in an arched, zigzag or wave form.

17. Disc-shaped cotton pad according to one of the claims 9 to 16,
**characterised in that**
the pattern of the wide striae (23) on one upper side is different from the pattern of the wide striae on the other upper side.

18. Disc-shaped cotton pad according to one of the preceding claims,
**characterised in that**
the two upper sides have different colours.

19. Disc-shaped cotton pad according to one of the preceding claims,
**characterised in that**
at least one of the upper sides is provided with markings.

20. Disc-shaped cotton pad according to one of the preceding claims,
**characterised in that**
the intermediate distances and depths of the fine striae (2, 22) on one upper side are different from the intermediate distances and depths of the fine striae (4, 10) on the other upper side.

## Revendications

1. Tampon constitué de préférence de fibres de coton blanchies en particulier à usage cosmétique ou hygiénique, lequel comporte
- de fines stries (3, 9) parallèles, de 0,1 à 0,2 mm de profondeur et avec un écartement entre les stries compris entre 0,5 et 1, 5 mm, formées par aiguilletage hydraulique au moins sur une face supérieure,
**caractérisé en ce que**
- des nervures (1, 7) formées par un second processus subséquent d'aiguilletage hydraulique sont réalisées au moins sur une des faces supérieures comportant les fines stries (3, 9),
- les nervures (1, 7) ont une largeur comprise environ entre 1,0 et 10,0 mm, une hauteur comprise entre 0,4 et 2,5 mm et un écartement entre les nervures compris entre 2 et 10 mm,
- sachant que les nervures (1, 7) et les zones enfoncées (2, 8) entre les nervures adjacentes (1, 7) comportent les fines stries (3, 9) situées sur la face supérieure .

2. Tampon selon la revendication 1,
**caractérisé en ce que**
les deux faces supérieures comportent les fines stries (3, 4) et seule une des faces supérieures comporte les nervures (1).

3. Tampon selon la revendication 1,
**caractérisé en ce que**
des nervures (7, 11) sont réalisées sur les deux faces supérieures, les nervures (7) de l'une des faces supérieures pouvant être différentes des nervures (11) de l'autre face supérieure en ce qui concerne la forme et le tracé.

4. Tampon selon l'une des revendications précédentes,
**caractérisé en ce que**
les nervures (1a, 1b; 1d; 1e) ont un tracé continu ou discontinu, rectiligne, ondulé, parallèle ou croisé.

5. Tampon constitué de préférence de fibres de coton blanchies en particulier à usage cosmétique ou hygiénique, lequel comporte
- de fines stries (3, 9) parallèles, de 0,1 à 0,2 mm de profondeur et avec un écartement entre les stries compris entre 0,5 et 1, 5 mm, formées par aiguilletage hydraulique au moins sur une face supérieure,
**caractérisé en ce que**
des alvéoles (12) arrondies ou polygonales formées par un second processus subséquent d'aiguilletage hydraulique, d'une profondeur comprise entre 0,5 et 2,5 mm et d'un diamètre compris entre 3 et 10 mm, sont réalisées au moins sur une des faces supérieures comportant les fines stries.

6. Tampon selon la revendication 5,
**caractérisé en ce que**
les deux faces supérieures comportent les fines stries et seule une des faces supérieures comporte les alvéoles (12).

7. Tampon selon la revendication 5,
**caractérisé en ce que**
des alvéoles (12) de nature différente sont réalisés sur les deux faces supérieures.

8. Tampon selon la revendication 5,
**caractérisé en ce que**
de fines stries (3) et des nervures (1, 7) sont réalisées sur l'autre face supérieure dépourvue d'alvéoles (12) selon l'une des revendications 1 ou 4.

9. Tampon constitué de préférence de fibres de coton blanchies en particulier à usage cosmétique ou hygiénique, lequel comporte
- de fines stries (22) parallèles, de 0,1 à 0,2 mm de profondeur et avec un écartement entre les stries compris entre 0,5 et 0,7 mm, formées au moins sur une face supérieure (21) par aiguilletage hydraulique,
**caractérisé en ce que**
- de larges stries (23) supplémentaires, d'une profondeur comprise entre 0,3 et 0,8 mm et avec un écartement entre les stries compris entre 9 et 15 mm, formées par un second processus subséquent d'aiguilletage hydraulique sont réalisées au moins sur une des faces supérieures (21) comportant les fines stries (22).

10. Tampon selon la revendication 9,
**caractérisé en ce que**
chaque large strie (23) est formée par un large jet d'eau sous pression et comporte un fond de strie (31) prononcé.

11. Tampon selon la revendication 9,
**caractérisé en ce que**
chaque large strie (23) est formée par deux jets d'eau sous pression prévus l'un à côté de l'autre et comporte dans le fond de strie deux creux latéraux (28,29) ainsi qu'une bosse médiane (30).

12. Tampon selon l'une des revendications 9 à 11,
**caractérisé en ce que**
les deux faces supérieures comportent les fines stries (22) et seule une des faces supérieures comporte les larges stries (23).

13. Tampon selon l'une des revendications 9 à 11,
**caractérisé en ce que**
les deux faces supérieures sont pourvues de larges stries (23) continues ou discontinues, lesquelles ont des écartements intermédiaires distincts ou sont disposées de manière décalée les unes par rapport aux autres.

14. Tampon selon l'une des revendications 9 à 13,
**caractérisé en ce que**
les larges stries (23) sont parallèles les unes par rapport aux autres et par rapport aux fines stries (22).

15. Tampon selon l'une des revendications 9 à 14,
**caractérisé en ce que**
les larges stries (23) sont disposées dans deux groupes de stries croisés selon des angles respectivement aigus par rapport aux fines stries (22).

16. Tampon selon l'une des revendications 9 à 15,
**caractérisé en ce que**
les larges stries (23) ont un tracé arqué, en zigzag ou ondulé.

17. Tampon selon l'une des revendications 9 à 16,
**caractérisé en ce que**
le motif des larges stries (23) sur l'une des faces supérieures est différent de celui des larges stries sur l'autre face supérieure.

18. Tampon selon l'une des revendications précédentes,
**caractérisé en ce que**
les deux faces supérieures ont des couleurs différentes.

19. Tampon selon l'une des revendications précédentes,
**caractérisé en ce qu'**
au moins une des faces supérieures est pourvue de repères.

20. Tampon selon l'une des revendications précédentes,
**caractérisé en ce que**
les écartements intermédiaires et profondeurs des fines stries (2, 22) sur l'une des faces supérieures sont différents des écartements intermédiaires et profondeurs des fines stries (4, 10) sur l'autre face supérieure.
